# EUROPÄISCHE PATENTANMELDUNG

(11) **EP 4 691 372 A1**
(43) Veröffentlichungstag der Anmeldung: **11.02.2026**
(21) Anmeldenummer: 24193104.7
(22) Anmeldetag: 06.08.2024
(51) Int. Cl.: A61B 6/04, A61B 6/50

(54) **COMPUTERIMPLEMENTIERTES VERFAHREN ZUM BETRIEB EINER MAMMOGRAPHIEEINRICHTUNG UND MAMMOGRAPHIEEINRICHTUNG**

(71) Anmelder: Siemens Healthineers AG, 91301 Forchheim (DE)
(72) Erfinder: Radicke, Marcus, 90587 Veitsbronn (DE)
(74) Vertreter: Siemens Healthineers Patent Attorneys

(57) **Zusammenfassung**

Die Erfindung betrifft ein computerimplementiertes Verfahren zum Betrieb einer Mammographieeinrichtung (12), welche aufweist:
- einen Objektträger (15) für ein aufzunehmendes Untersuchungsobjekt,
- eine dem Objektträger (15) zugeordnete Kompressionseinrichtung mit wenigstens einer beweglichen Kompressionsplatte (18), der ein Aktor (20) zur Bewegung der Kompressionsplatte (18) zugeordnet ist,
- wenigstens ein Positionserfassungsmittel (21) zur Erfassung eines Positionswerts, der die aktuelle Position der beweglichen Kompressionsplatte (18) beschreibt, und wenigstens ein Krafterfassungsmittel (22) zur Erfassung eines Kraftwerts, der die durch die Kompressionsplatte (18) ausgeübte Kraft beschreibt, und
- eine Steuereinrichtung (23), die zum automatischen Einstellen einer Zielkompressionsstellung der beweglichen Kompressionsplatte (18) für ein Untersuchungsobjekt durch Ansteuerung des Aktors (20) in Abhängigkeit von dem Positionswert, dem Kraftwert und einem vorgegebenen Schwellwert ausgebildet ist, wobei das Verfahren folgende Schritte zum automatischen Einstellen einer Kompression des Untersuchungsobjekts für einen Aufnahmevorgang aufweist:
- Ermitteln eines Referenzflächenwerts, der eine angenommene Kontaktfläche zwischen dem komprimierten Untersuchungsobjekt und der beweglichen Kompressionsplatte (18) in der Zielkompressionsstellung beschreibt, unter Verwendung des Kraftwerts und des Positionswerts zu wenigstens einem Referenzzeitpunkt,
- Komprimieren des Untersuchungsobjekts durch Ansteuerung des Aktors (20), bis eine Abbruchbedingung erfüllt ist, wobei die Abbruchbedingung das Erreichen eines vorgegebenen Solldrucks durch den Kraftwert bezogen auf den Referenzflächenwert beschreibt.

## Beschreibung

Die Erfindung betrifft ein computerimplementiertes Verfahren zum Betrieb einer Mammographieeinrichtung, welche aufweist:
- einen Objektträger für ein aufzunehmendes Untersuchungsobjekt, insbesondere eine Brust,
- eine dem Objektträger zugeordnete Kompressionseinrichtung mit wenigstens einer beweglichen Kompressionsplatte, der ein Aktor zur Bewegung der Kompressionsplatte zugeordnet ist,
- wenigstens ein Positionserfassungsmittel zur Erfassung eines Positionswerts, der die aktuelle Position der beweglichen Kompressionsplatte beschreibt,
- wenigstens ein Krafterfassungsmittel zur Erfassung eines Kraftwerts, der die durch die Kompressionsplatte ausgeübte Kraft beschreibt, und
- eine Steuereinrichtung, die zum automatischen Einstellen einer Zielkompressionsstellung der beweglichen Kompressionsplatte für ein Untersuchungsobjekt durch Ansteuerung des Aktors in Abhängigkeit von dem Positionswert, dem Kraftwert und einem vorgegebenen Schwellwert ausgebildet ist.

Daneben betrifft die Erfindung auch eine Mammographieeinrichtung.

Mammographieeinrichtungen sind Röntgenbildgebungseinrichtungen, die üblicherweise einen Objektträger umfassen, auf dem ein Untersuchungsobjekt, insbesondere eine Brust (Mamma), zur Aufnahme mit einer Aufnahmeanordnung platziert werden kann. Um möglichst wenig überlappendes Gewebe zu durchstrahlen, wird das Untersuchungsobjekt mittels einer Kompressionseinrichtung zwischen dem Objektträger und einer beweglichen Kompressionsplatte (englisch "compression paddle") komprimiert. Der Objektträger kann hierbei der Röntgendetektor sein oder diesen umfassen.

Die Kompression in der Mammographie bringt eine Vielzahl von Vorteilen mit sich, beispielsweise die Reduzierung von Bewegungen, von Gewebeüberlapp und der durchschnittlichen Röntgendosis, die bessere Ausnutzung der räumlichen Auflösung des Detektors, die Reduzierung von Streustrahlung und Strahlaufhärtung sowie eine bessere Nutzung des Dynamikbereichs des Detektors aufgrund der gleichmäßigen Dicke. Nichtsdestotrotz ist ein Problem, das eine zu starke Kompression eingestellt werden könnte, die zu Unwohlbefinden und gegebenenfalls sogar Schmerzen führen kann.

In einem White Paper von Johannes Georg Korporaal, "Breast Compression with SoftSpeed and OpComp", Siemens Healthcare GmbH, 2017, wird ein Lösungsansatz beschrieben, bei dem die Dickenänderung der Brust über die Zeit, die zu einer wirkenden Kompressionskraft in Beziehung gesetzt werden kann, analysiert wird und ein Schwellwert für den Gradienten der Dickenänderung über die Zeit festgelegt wird, bei dem die Bewegung der Kompressionsplatte angehalten wird und als Zielkompressionsstellung verwendet wird.

Problematisch hierbei ist jedoch, dass individuelle Unterschiede in den Brüsten existieren und daher bei Verwendung eines Schwellwerts für den Gradienten unterschiedliche Empfindungen der vorliegenden Kompression auftreten können. Hieran kann auch eine Nachjustage durch eine Bedienperson nicht immer etwas ändern, da diese ebenso eine perfekte Einstellung nicht ersehen kann.

Der Erfindung liegt daher die Aufgabe zugrunde, eine verbesserten Komfort und ein verbessertes Auffinden einer geeigneten Zielkompressionsstellung erlaubende Möglichkeit zur Steuerung der automatischen Bewegung einer Kompressionsplatte einer Mammographieeinrichtung anzugeben.

Diese Aufgabe wird gelöst durch ein computerimplementiertes Verfahren und eine Mammographieeinrichtung gemäß den unabhängigen Patentansprüchen. Vorteilhafte Weiterbildungen ergeben sich aus den abhängigen Ansprüchen.

Bei einem Verfahren der eingangs genannten Art ist erfindungsgemäß vorgesehen, dass das Verfahren folgende Schritte zum automatischen Einstellen einer Kompression des Untersuchungsobjekts für einen Aufnahmevorgang aufweist:
- Ermitteln eines Referenzflächenwerts, der eine angenommene Kontaktfläche zwischen dem komprimierten Untersuchungsobjekt und der beweglichen Kompressionsplatte in der Zielkompressionsstellung beschreibt, unter Verwendung des Kraftwerts und des Positionswerts zu wenigstens einem Referenzzeitpunkt, und
- Komprimieren des Untersuchungsobjekts durch Ansteuerung des Aktors, bis eine Abbruchbedingung erfüllt ist, wobei die Abbruchbedingung das Erreichen eines vorgegebenen Solldrucks durch den Kraftwert bezogen auf den Referenzflächenwert beschreibt.

Es wird vorgeschlagen, hinsichtlich des Schwellwerts nicht länger auf die Kraft selbst bzw. einen Gradienten abzustellen, sondern individuelle Eigenschaften des Untersuchungsobjekts, insbesondere der Brust, heranzuziehen, um einen allgemein zur Bildgebung sinnvollen, aber noch hinreichend komfortablen Solldruck auf das Untersuchungsobjekt, insbesondere die Brust, zu erreichen. Dazu wird vorgeschlagen, die Kontaktfläche abzuschätzen, die dem Solldruck auszusetzen ist, in dem während des automatischen Betriebs der Kompressionsplatte aufgenommene Messdaten herangezogen werden. Ist diese Kontaktfläche als der Referenzflächenwert erst abgeschätzt, lässt sich aus der aktuell anliegenden Kompressionskraft unter Annahme dieser Kontaktfläche der aktuell anliegende Druck ermitteln bzw. aus dem Solldruck ein vorgangsspezifischer Schwellwert ermitteln, um auf den Solldruck regeln zu können.

Konkrete Möglichkeiten der vorliegenden Erfindung können also vorsehen, dass zur Überprüfung der Erfüllung der Abbruchbedingung
- ein den aktuellen Druck auf das Untersuchungsobjekt beschreibender aktueller Druckwert aus dem aktuellen Kraftwert und dem Referenzflächenwert ermittelt wird, und
- der aktuelle Druckwert mit dem als der Solldruck bereitgestellten Schwellwert verglichen wird, wobei die Abbruchbedingung erfüllt ist, wenn der Druckwert größer oder gleich dem Schwellwert ist,
   oder
- ein vorgangsspezifischer Schwellwert für den Kraftwert aus dem Solldruck und dem Referenzflächenwert ermittelt wird und
- der aktuelle Kraftwert mit dem Schwellwert verglichen wird, wobei die Abbruchbedingung erfüllt ist, wenn der Kraftwert größer oder gleich dem Schwellwert ist.

Mit anderen Worten ist es zum einen möglich, den Kraftwert auf einen entsprechenden Druckwert umzurechnen, der mit dem als Solldruck bereitgestellten Schwellwert verglichen werden kann, und zum anderen, einen letztlich individualisierten, aus dem Solldruck ermittelten Schwellwert für den Kraftwert bereitzustellen. Auf diese Weise kann mithin speziell auf das Untersuchungsobjekt, insbesondere die Brust, die im aktuellen Aufnahmevorgang aufgenommen werden soll, abgestellt werden und somit ein hoher Komfort und eine hohe Bildqualität erreicht werden. Mit anderen Worten wird eine Individualisierung des bekannten Vorgehens vorgeschlagen, die wenigstens die Größe des Untersuchungsobjekts, insbesondere also die Brustgröße, berücksichtigt. Auf diese Weise können automatische Einstellvorgänge für die Kompressionseinrichtung in ihrer Akzeptanz und somit auch in ihrem Einsatz deutlich erhöht werden.

Dabei lässt sich das Verfahren problemlos an gängigen Mammographieeinrichtungen umsetzen, die meist ohnehin das wenigstens eine Krafterfassungsmittel und das Positionserfassungsmittel aufweisen. Das Positionserfassungsmittel kann dabei durch den Aktor selbst bereitgestellt werden, der mithin eine Geberfunktionalität aufweisen kann oder aber zumindest einen Geber umfassen kann, sodass die Bewegung der Kompressionsplatte und somit auch ihre aktuelle Position nachvollzogen werden kann. Der Objektträger kann dabei durch den Röntgendetektor der Mammographieeinrichtung gebildet werden oder den Röntgendetektor wenigstens umfassen. Die zugehörige Röntgenquelle, die die Aufnahmeanordnung vervollständigt, kann die Kompressionsplatte durchstrahlend angeordnet werden. Hierbei kann die Mammographieeinrichtung sowohl zur zweidimensionalen, als auch zur dreidimensionalen Bildgebung, insbesondere zur Tomosynthese, ausgebildet sein.

Die Nutzung des Positionserfassungsmittel und des wenigstens einen Krafterfassungsmittels erlaubt es, auf weitere Erfassungsmittel zur Erfassung der Größe des Untersuchungsobjekts zu verzichten. Insbesondere sind bei Anwendung des hier beschriebenen Verfahrens keinerlei Kameras oder anderweitig datenschutzrechtlich relevante oder bedenkliche Sensoren notwendig.

Es ist vorgesehen, dass ein geeigneter Solldruck vorgegeben wird, der im Abbruchkriterium verwendet wird. Während es hierbei grundsätzlich denkbar ist, einen allgemeinen Kompromiss für unterschiedliche Anwendungsfälle heranzuziehen, mithin einen festen vorgegebenen Solldruck für verschiedene Anwendungsfälle zu verwenden, ist es erfindungsgemäß bevorzugt, auch bezüglich des Solldrucks auf den einzelnen Aufnahmevorgang hin Anpassungen vorzunehmen, um eine weitere Verbesserung hinsichtlich der Bildqualität und des Komforts zu erreichen.

So sieht eine bevorzugte Weiterbildung der vorliegenden Erfindung vor, dass der Solldruck in Abhängigkeit wenigstens einer den Untersuchungsvorgang, insbesondere die Zieldimensionalität des zu ermittelnden Bilddatensatzes, beschreibenden Untersuchungsinformation vorgegeben wird. Beispielsweise kann die konkrete Modalität genauso berücksichtigt werden wie Einstellungen der Aufnahmeanordnung für den Aufnahmevorgang, insbesondere was die Röntgendosis angeht. In besonders vorteilhafter Weiterbildung der vorliegenden Erfindung ist jedoch vorgesehen, dass der Solldruck für eine dreidimensionale Aufnahme niedriger vorgegeben wird als für eine zweidimensionale Aufnahme. Untersuchungen haben gezeigt, dass für dreidimensionale Aufnahmevorgänge, insbesondere die Tomosynthese, niedrigere Kompression angesetzt werden kann, ohne diagnostische Leistungsfähigkeit zu verlieren. Dies liegt darin begründet, dass Bilddaten aus unterschiedlichen Richtungen gewonnen werden und sodann in der Rekonstruktion kombiniert werden, sodass eine robustere Ermittlung des letztendlichen Bilddatensatzes erfolgt. Es wird nun vorgeschlagen, dies zu berücksichtigen, um bei Aufrechterhaltung der notwendigen Bildqualität, insbesondere hinsichtlich der diagnostischen Eignung, mehr Komfort für den jeweiligen Patienten zuzulassen.

In besonders vorteilhaften Ausführungsbeispielen kann zudem vorgesehen sein, dass in Abhängigkeit des Verlaufs des Kraftwerts über den Positionswert wenigstens eine Eigenschaftsinformation des Untersuchungsobjekts ermittelt wird und der Solldruck in Abhängigkeit der Eigenschaftsinformation, insbesondere gemäß einer ersten Zuordnungsvorschrift, vorgegeben wird. Hierbei kann mit besonderem Vorteil neben dem Verlauf auch der Referenzflächenwert in die Ermittlung der Eigenschaftsinformation eingehen. Mit anderen Worten kann die Messung der Kraftänderung über die Positionsänderung (Wegänderung) eine Rückmeldung über die elastischen Eigenschaften des Untersuchungsobjekts, insbesondere der Brust, und damit über dessen Zusammensetzung geben. Dies gilt insbesondere dann, wenn auch die bekannte Brustfläche, also der Referenzflächenwert, mit einbezogen wird. Beispielsweise können dann Rückschlüsse über die Zusammensetzung einer aufzunehmenden Brust, beispielsweise als das Verhältnis von Drüsengewebe zu Fettgewebe, gezogen werden und es kann der Solldruck als Zieldruck für die individuelle Brustzusammensetzung gewählt werden. Konkret kann eine erste Zuordnungsvorschrift genutzt werden, um anhand des Verlaufs und vorzugsweise auch des Referenzflächenwerts einen geeigneten Solldruck zu bestimmen.

Die erste Zuordnungsvorschrift ist bevorzugt eine Look-Up-Tabelle (LUT). Werden auch Untersuchungsinformationen berücksichtigt, können verschiedene Look-up-Tabellen für verschiedene Untersuchungsinformationen, beispielsweise eine für zweidimensionale Aufnahmen und eine für dreidimensionale Aufnahmen, bereitgestellt werden. Besonders bevorzugt ist es jedoch, eine gemeinsame Look-up-Tabelle für die Untersuchungsinformationen und die Eigenschaftsinformationen vorzusehen, um auf einfache Art und Weise den geeigneten Solldruck bestimmen zu können.

Eine bevorzugte Ausgestaltung der vorliegenden Erfindung kann vorsehen, dass als Referenzzeitpunkt der Erstkontaktzeitpunkt der Kompressionsplatte mit dem Untersuchungsobjekt mittels wenigstens eines des wenigstens einen Krafterfassungsmittels detektiert wird, wobei aus dem Positionswert zum Referenzzeitpunkt ein Referenzhöhenwert des Untersuchungsobjekts als Abstand zum Objektträger ermittelt wird und aus dem Referenzhöhenwert der Referenzflächenwert ermittelt wird. Im Falle einer Brust kann der so entstehende Referenzhöhenwert als eine anfängliche Brustdicke zu Beginn der Kompression aufgefasst werden. Hierbei kann mittels wenigstens eines des wenigstens einen Krafterfassungsmittels, insbesondere eines Kraftsensor, erfasst werden, dass die Kompressionsplatte das Untersuchungsobjekt kontaktiert, da dieses der Kompression selbstverständlich einen Widerstand entgegengesetzt, der sich in einem Anstieg der Kraft äußert. Als günstiger Detektionswert zur Detektion des Erstkontaktzeitpunkts hat sich beispielsweise fünf Newton erwiesen. Aufgrund des Positionswerts ist bekannt, wo sich die Kompressionsplatte relativ zu dem Objektträger, an dem das Untersuchungsobjekt ebenso anliegt, befindet, sodass der Abstand von Kompressionsplatte zu Objektträger dem Referenzhöhenwert, also der anfänglichen Untersuchungsobjektdicke, entspricht.

Zur konkreten Ermittlung des Referenzflächenwerts aus dem Referenzhöhenwert kann dann vorgesehen sein, dass eine statistisch ermittelte zweite Zuordnungsvorschrift, die einem Referenzhöhenwert eine statistisch wahrscheinlichste Referenzfläche als Referenzflächenwert zuordnet, insbesondere eine Look-Up-Tabelle, verwendet wird. Mit anderen Worten kann der Referenzhöhenwert als Basis genutzt werden, um eine statistisch typische Kontaktfläche des Untersuchungsobjekts mit der Kompressionsplatte in der Zielkompressionsstellung festzustellen. Hierbei können beispielsweise Untersuchungsreihen, mithin Versuche, durchgeführt werden, in denen gezielt auf die beschriebene Art der Referenzhöhenwert und ein zugeordneter Referenzflächenwert ermittelt werden. Zur Ermittlung des Referenzflächenwerts für statistische Messreihen, aus denen die zweite Zuordnungsvorschrift hergeleitet werden kann, existieren verschiedene Möglichkeiten, die beispielsweise wenigstens teilweise auf der Annahme fußen können, dass das Volumen des Untersuchungsobjekts, insbesondere der Brust, konstant bleibt. Beispielsweise können klinische Studien verwendet werden, in denen eine Kamera auf die Brust gerichtet wird. Möglich ist es auch, mit der Mammographieeinrichtung aufgenommene Bilddaten auszuwerten, um die Kontaktfläche der Brust (oder sonstigen Untersuchungsobjekts) mit der Kompressionsplatte, insbesondere unter der Annahme konstanten Volumens, zu ermitteln. Solche Messreihen können statistisch ausgewertet werden, um festzustellen, welche Kontaktfläche als Referenzflächenwert bei einem bestimmten Referenzhöhenwert, also einer bestimmten anfänglichen Untersuchungsobjektdicke, am wahrscheinlichsten ist. Diese wahrscheinlichste Kontaktfläche kann dann angenommen werden, um den Solldruck geeignet verarbeiten zu können. Durch die Nutzung solcher statistische Analysen ausgehend von der einfach möglichen und meist ohnehin bereits durchgeführten Detektion des Erstkontaktzeitpunkts, an dem sich also eine erste Kontaktfläche zum Untersuchungsobjekt aufbaut, ist eine aufwandsarme Abschätzung der Brustgröße in Form des Referenzflächenwerts möglich.

Dabei kann auch im Rahmen der vorliegenden Erfindung, wie dies im Stand der Technik grundsätzlich bereits vorgeschlagen wurde, vorgesehen sein, dass die Ansteuerung des Aktors in zwei Betriebsphasen erfolgt, wobei die Kompressionsplatte in der ersten Betriebsphase an das Untersuchungsobjekt, insbesondere mit konstanter Geschwindigkeit, herangefahren wird, und ab dem Referenzzeitpunkt in einer zweiten Betriebsphase mit insbesondere über die Zeit steigender Kompressionskraft zur Kompression des Untersuchungsobjekts weiterbewegt wird. In diesem Fall dient die Detektion des Erstkontaktzeitpunkts mithin einem doppelten Zweck, nämlich zum einen dem Phasenübergang in der Ansteuerung der Bewegung der Kompressionsplatte, zum anderen der Nutzung als Referenzzeitpunkt, zu dem der Referenzhöhenwert ermittelt wird.

Hinsichtlich des Krafterfassungsmittels kann vorgesehen sein, dass wenigstens eines des wenigstens einen Krafterfassungsmittels als Kraftsensor die über die Kompressionsplatte ausgeübte Kompressionskraft unmittelbar misst und/oder wenigstens eines des wenigstens einen Kraftferfassungsmittels eine Zeit, insbesondere ab einem Erstkontaktzeitpunkt und/oder der zweiten Betriebsphase, misst und der Kraftwert aufgrund eines bekannten Ansteuerungsverlaufs mit der Zeit ermittelt wird. Im erstgenannten Fall wird somit die von der Kompressionsplatte ausgeübte Kompressionskraft unmittelbar und auf besonders verlässliche Art und Weise mittels des Kraftsensors erfasst. Jedoch ist auch der zweite Fall im Rahmen der vorliegenden Erfindung (weiterhin) anwendbar, wenn ein Ansteuerungsverlauf existiert, der anzeigt, wie die Kompressionskraft, insbesondere in der zweiten Betriebsphase, über die Zeit erhöht wird. Im Stand der Technik sind bereits Mammographieeinrichtungen bekannt, in denen sich die Kompressionskraft in der zweiten Betriebsphase linear mit der Zeit erhöht, sodass die Zeit mithin proportional zur Kompressionskraft ist.

In einer besonders bevorzugten Ausgestaltung der vorliegenden Erfindung kann vorgesehen sein, dass die Mammographieeinrichtung ferner eine Anzeigeeinrichtung aufweist, die von der Steuereinrichtung zur Visualisierung des ermittelten Referenzflächenwerts in Relation zu der Größe der Kompressionsplatte und/oder des Objektträgers angesteuert wird. Dem liegt der Gedanke zugrunde, dass es sich bei der Ermittlung des Referenzflächenwerts lediglich um eine Abschätzung handelt, insbesondere wenn eine statistische Analyse als Grundlage herangezogen wird. Das bedeutet, es liegt eine individuelle Fehlerbehaftung vor. Mithin wird vorgeschlagen, die der automatischen Einstellung zugrunde liegende angenommene Brustgröße, also den Referenzflächenwert, für eine Bedienperson zu visualisieren. Dies erfolgt bevorzugt unmittelbar mit dessen Ermittlung, beispielsweise zum Erstkontaktzeitpunkt. Die Bedienperson kann dann durch Beobachtung der realen Situation schnell feststellen, ob die Abschätzung korrekt ist, und gegebenenfalls eingreifen und/oder nach Erreichen der Zielkompressionsstellung manuell noch korrigieren.

Konkret kann beispielsweise vorgesehen sein, dass zur Visualisierung ein die Größe der Kompressionsplatte und/oder des Objektträgers anzeigendes Rechteck gemäß der Form des Untersuchungsobjekts teilweise, den relativen Anteil des Referenzflächenwerts anzeigend, aufgefüllt wird, insbesondere zumindest grob der Form des Untersuchungsobjekts folgend und/oder von einer Seite des Rechtecks ausgehend. Auf diese Weise ist eine äußerst intuitive Darstellung gegeben, die unmittelbar zum tatsächlichen Geschehen am Objektträger in Verbindung gesetzt werden kann.

Besonders zweckmäßig ist es ferner, wenn, insbesondere für einen manuellen Einstellvorgang, zusätzlich eine Information angezeigt wird, die beschreibt, wie die aktuelle Kompression für kleinere und/oder größere Untersuchungsobjekte anzupassen wäre, um den Solldruck zu erhalten. Mit anderen Worten kann zusätzlich ein Hinweis ausgegeben werden, ob bei einer größeren/kleineren Brustgröße bzw. allgemein Untersuchungsobjektgröße die Kompressionskraft und damit der Druck erhöht oder erniedrigt werden sollte. Auf diese Weise wird neben der intuitiven Wiedergabe des Ist-Bezugs bei Erkennen einer Abweichung von der Realität unmittelbar die Möglichkeit gegeben, manuell auf korrekte Weise einzugreifen.

In einer Weiterbildung der Erfindung kann ferner vorgesehen sein, dass die Steuereinrichtung ferner dazu ausgebildet ist, für den aktuellen Kraftwert und/oder wenigstens eine Referenzkraft zu visualisieren, bei welcher relativen Fläche des Untersuchungsobjekts zu der Fläche der Kompressionsplatte der Solldruck gegeben ist. Es kann also, insbesondere bei manueller Einstellung der Kompressionskraft, die Kontaktfläche visualisiert werden, die aus Sicht der Kompression, also bezüglich des Solldrucks, ideal zu der aktuellen Kompressionskraft passen würde. Bei Erhöhung der aktuellen Kompressionskraft würde somit die beispielsweise als das Untersuchungsobjekt, insbesondere die Brust, visualisierte Kontaktfläche größer. Somit wird der Bedienperson eine patientenindividualisierte Rückmeldung gegeben, die eine verbesserte manuelle Einstellung der Kompressionskraft ermöglicht.

Diese Visualisierung bei der manuellen Einstellung der Kompressionskraft kann auch unabhängig von der oben beschriebenen Steuerung der automatischen Einstellung, insbesondere unter Verwendung des Referenzflächenwerts, sinnvoll sein. Es ergäbe sich dann ein Verfahren der eingangs genannten Art, wobei zur Unterstützung der manuellen Einstellung einer Zielkompression aus dem aktuellen Kraftwert und einem vorgegebenen Solldruck eine Referenzkontaktfläche ermittelt wird und die relative Größe der Referenzkontaktfläche zu der Fläche der Kompressionsplatte an einer Anzeigeeinrichtung der Mammographieeinrichtung visualisiert wird. Denkbar ist in diesem Zusammenhang ferner auch, eine grundsätzliche, permanente Visualisierung für einen Minimalwert des Kraftwerts und einen Maximalwert des Kraftwerts sowie ggf. wenigstens einen Zwischenwert an einer Skala, die den aktuellen Kraftwert anzeigt, vorzusehen. Hierbei können die oben bereits genannten Anzeigekonzepte, insbesondere das mit steigender Größe durch die Brust immer weiter aufgefüllte, die Kompressionsplatte symbolisierende Rechteck, angewendet werden.

Neben dem Verfahren betrifft die vorliegende Erfindung auch eine Mammographieeinrichtung, welche aufweist:
- einen Objektträger für ein aufzunehmendes Untersuchungsobjekt, insbesondere eine Brust,
- eine dem Objektträger zugeordnete Kompressionseinrichtung mit wenigstens einer beweglichen Kompressionsplatte, der ein Aktor zur Bewegung der Kompressionsplatte zugeordnet ist,
- wenigstens ein Positionserfassungsmittel zur Erfassung eines Positionswerts, der die aktuelle Position der beweglichen Kompressionsplatte beschreibt,
- wenigstens ein Krafterfassungsmittel zur Erfassung eines Kraftwerts, der die durch die Kompressionsplatte ausgeübte Kraft beschreibt, und
- eine Steuereinrichtung, die zum automatischen Einstellen einer Zielkompressionsstellung der beweglichen Kompressionsplatte für ein Untersuchungsobjekt durch Ansteuerung des Aktors in Abhängigkeit von dem Positionswert, dem Kraftwert und einem vorgegebenen Schwellwert ausgebildet ist,
wobei die Steuereinrichtung zum automatischen Einstellen einer Kompression des Untersuchungsobjekts für einen Aufnahmevorgang aufweist:
- eine Schnittstelle zum Empfang des Kraftwerts und des Positionswerts,
- eine Ermittlungseinheit zum Ermitteln eines Referenzflächenwerts, der eine angenommene Kontaktfläche zwischen dem komprimierten Untersuchungsobjekt und der beweglichen Kompressionsplatte in der Zielkompressionsstellung beschreibt, unter Verwendung des Kraftwerts und des Positionswerts zu wenigstens einem Referenzzeitpunkt, und
- eine Steuereinheit zur Komprimieren des Untersuchungsobjekts durch Ansteuerung des Aktors, bis eine Abbruchbedingung erfüllt ist, wobei die Abbruchbedingung das Erreichen eines vorgegebenen Solldrucks durch den Kraftwert bezogen auf den Referenzflächenwert beschreibt.

Sämtliche Ausführungen bezüglich des erfindungsgemäßen Verfahrens lassen sich analog auf die erfindungsgemäße Mammographieeinrichtung übertragen und umgekehrt, sodass auch mit der Mammographieeinrichtung die bereits genannten Vorteile erhalten werden können.

Die Steuereinrichtung kann konkret zur Durchführung eines erfindungsgemäßen Verfahrens ausgebildet sein. Sie weist wenigstens einen Prozessor und wenigstens ein Speichermittel auf, wobei in dem Speichermittel beispielsweise die bereits beschriebenen Look-up-Tabellen (erste und zweite Zuordnungsvorschrift) gespeichert sein können. Durch Hardware und/oder Software werden Funktionseinheiten bereitgestellt, um den Betrieb der Mammographieeinrichtung im Allgemeinen zu steuern und insbesondere Schritte des erfindungsgemäßen Verfahrens ausführen zu können. Dabei können neben der bereits genannten Ermittlungseinheit und der Steuereinheit auch weitere Funktionseinheiten vorgesehen werden, um weitere Ausgestaltungen des Verfahrens umzusetzen. Beispielsweise kann die Steuereinrichtung auch eine Vorgabeeinheit aufweisen, um den Solldruck für einen jeweiligen Anwendungsfall und Patienten zu ermitteln, insbesondere unter Nutzung der ersten Zuordnungsvorschrift. Ferner kann eine Triggereinheit vorgesehen sein, um den Erstkontaktzeitpunkt als Referenzzeitpunkt zu detektieren. Auch eine Darstellungseinheit zur Ansteuerung der Anzeigeeinrichtung, insbesondere zur Visualisierung des Referenzflächenwerts und/oder der Referenzkontaktfläche bei manueller Einstellung, kann vorgesehen sein.

Das erfindungsgemäße Verfahren kann auch als Computerprogramm umgesetzt werden. Das Computerprogramm weist dann Programmmittel auf, welche derart ausgestaltet sind, dass dann, wenn das Computerprogramm in einer Steuereinrichtung einer Mammographieeinrichtung ausgeführt wird, diese veranlasst wird, die Schritte eines erfindungsgemäßen Verfahrens auszuführen. Das Computerprogramm kann auf einem elektronisch lesbaren Datenträger gespeichert sein, welcher mithin darauf gespeicherte Steuerinformationen umfasst, die wenigstens ein erfindungsgemäßes Computerprogramm umfassen und derart ausgestaltet sind, dass bei Verwendung des Datenträgers in einer Steuereinrichtung einer Mammographieeinrichtung diese ausgebildet wird, ein erfindungsgemäßes Verfahren durchzuführen.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: einen Ablaufplan eines Ausführungsbeispiels des erfindungsgemäßen Verfahrens,
- Fig. 2: eine erste mögliche Anzeige auf einer Anzeigeeinrichtung,
- Fig. 3: eine zweite mögliche Anzeige auf einer Anzeigeeinrichtung,
- Fig. 4: eine dritte mögliche Anzeige auf einer Anzeigeeinrichtung,
- Fig. 5: eine Prinzipskizze einer erfindungsgemäßen Mammographieeinrichtung, und
- Fig. 6: den funktionalen Aufbau einer Steuereinrichtung der Mammographieeinrichtung.

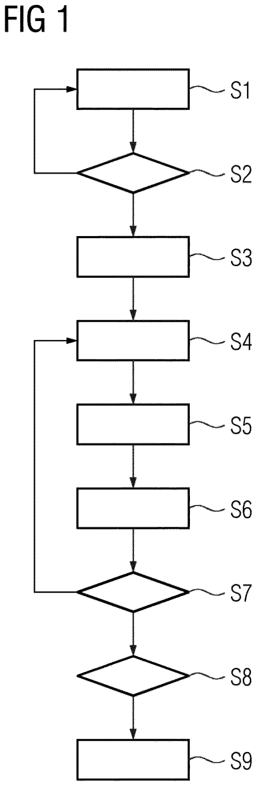

Fig. 1 zeigt einen Ablaufplan eines Ausführungsbeispiels des erfindungsgemäßen Verfahrens, wie es auf einer Steuereinrichtung einer Mammographieeinrichtung ausgeführt werden kann, um automatisch eine Kompression eines Untersuchungsobjekts, hier einer Brust, einzustellen, die einem Solldruck auf die Brust entspricht. Die Mammographieeinrichtung weist hierzu, wie grundsätzlich bekannt, einen Objektträger auf, der den Röntgendetektor umfassen oder durch diesen gebildet sein kann. Zu dem Röntgenstrahler der Mammographieeinrichtung hin folgt dem Objektträger eine Kompressionsplatte einer Kompressionseinrichtung, die mittels eines Aktors, der durch die Steuereinrichtung ansteuerbar ist, bewegbar ist, um Kompression auf die zwischen dem Objektträger und der Kompressionsplatte befindliche Brust auszuüben.

In einem Schritt S1 beginnt eine erste Betriebsphase zur Einstellung der Zielkompressionsstellung, in der die Kompressionsplatte, die zunächst noch von der Brust beabstandet war, an die Brust herangefahren wird. Dies kann mit konstanter Geschwindigkeit geschehen. In einem Schritt S2 wird anhand der Messdaten eines ersten Krafterfassungsmittels, beispielsweise eines Kraftsensors, überprüft, ob ein Erstkontaktzeitpunkt erreicht wurde, in dem die Kompressionsplatte die Brust berührt und eine erste Kontaktfläche aufbaut. Der detektierte Erstkontaktzeitpunkt bildet auch einen Referenzzeitpunkt.

Wie grundsätzlich bekannt, ist der Kompressionsplatte, insbesondere dem Aktor zugeordnet oder als Teil des Aktors, ein Positionserfassungsmittel zugeordnet, dessen Positionswert auch anzeigt, in welchem Abstand zu dem Objektträger sich die Kompressionsplatte befindet. Das Positionserfassungsmittel wird in einem Schritt S3 nun genutzt, um einen Referenzhöhenwert der Brust als den Abstand der Kompressionsplatte von dem Objektträger zu ermitteln, der zum Referenzzeitpunkt, also dem Erstkontaktzeitpunkt, vorliegt. Der Referenzhöhenwert wird wiederum genutzt, um daraus mittels einer zweiten Zuordnungsvorschrift, insbesondere einer Look-up-Tabelle, eine statistisch wahrscheinlichste Kontaktfläche der Kompressionsplatte mit der Brust in der Zielkompressionsstellung zu ermitteln. Hierzu wurde die zweite Zuordnungsvorschrift durch eine statistische Analyse entsprechender Messdaten, in denen Höhenwerte mit zugeordneten Kontaktflächen, die beispielsweise unter Annahme konstanten Volumens errechnet sind, ermittelt. Diese statistisch wahrscheinlichste Kontaktfläche bildet den Referenzflächenwert.

In einem Schritt S4 beginnt dann eine zweite Betriebsphase der Kompressionsplatte, in der diese mit steigender Kompressionskraft bewegt wird, um die Brust zu komprimieren. Hierbei wird mittels des ersten oder eines zweiten Krafterfassungsmittels auch ein Kraftwert, der die aktuell auf die Brust ausgeübte Kompressionskraft beschreibt, ermittelt. Dabei kann wiederum ein Kraftsensor verwendet werden, der beispielsweise in die Kompressionsplatte verbaut sein kann. Möglich ist es aber auch, einen festen Verlauf der Kompressionskraft mit der Zeit für die zweite Betriebsphase vorzugeben und die Zeit seit dem Erstkontaktzeitpunkt zu vermessen, sodass das zweite Krafterfassungsmittel ein Zeitgeber sein kann, der mittels des festen Verlaufs den Kraftwert liefert.

In einem Schritt S5 wird ein Solldruck, bei dem eine für den bevorstehenden Aufnahmevorgang hinreichende Bildqualität erlaubende Kompression der Brust vorliegt, die jedoch noch komfortabel für den Patienten bzw. die Patientin ist, ermittelt. Neben einer wenig bevorzugten, generellen Vorgabe des Solldrucks kann dieser abhängig von einer Untersuchungsinformation des Untersuchungsvorgangs und/oder einer Eigenschaftsinformation der Brust, die aus dem Verlauf des Kraftwerts hergeleitet werden kann, gewählt werden. Ist der Solldruck nur von einer Untersuchungsinformation, beispielsweise, ob eine zweidimensionale oder dreidimensionale Bildgebung erfolgen soll, abhängig, kann der Schritt S5 auch bereits früher im Verfahrensverlauf durchgeführt werden. Bei einer dreidimensionalen Bildgebung, beispielsweise einer Tomosynthese, kann der Solldruck, also die Kompression, geringer gewählt werden.

Besonders bevorzugt ist es jedoch, auch eine Eigenschaftsinformation einzubeziehen. Die Messung der Änderung der Kompressionskraft über die Änderung der Position (anhand des Kraftwerts und des Positionswerts) enthält - bei bekannter Referenzkontaktfläche - eine Information über die elastischen Eigenschaften der Brust und damit über die Zusammensetzung der Brust, insbesondere den Anteil von Drüsengewebe zu Fettgewebe. Unter Berücksichtigung solcher individueller Eigenschaften der Brust kann der Solldruck hervorragend so gewählt werden, dass kein zu starkes Unwohlsein oder sogar Schmerz aufgrund der Kompression auftritt. Eine erste Zuordnungsvorschrift, bevorzugt wiederum eine Look-Up-Tabelle, wird genutzt, um der Untersuchungsinformation und der Eigenschaftsinformation einen Solldruck zuzuordnen. Dabei kann eine stetige Aktualisierung des Solldrucks während der zweiten Betriebsphase mit der ständigen Aktualisierung bzw. Ergänzung der Eigenschaftsinformation erfolgen.

In einem Schritt S6 erfolgt zur Unterstützung einer Bedienperson, die die automatische Einstellung überwacht, eine Visualisierung der Referenzkontaktfläche auf einer Anzeigeeinrichtung der Mammographieeinrichtung. Diese wird relativ zur Fläche der Kompressionsplatte und/oder des Objektträgers gezeigt, sodass die Bedienperson einschätzen kann, ob die Referenzkontaktfläche aufgrund der statistisch wahrscheinlichsten Wahl korrekt abgeschätzt wurde oder ein individueller Abweichungsfall vorliegt. Bei einer starken Abweichung kann die automatische Einstellung abgebrochen und manuell weiter eingestellt werden, worauf im Folgenden noch näher eingegangen werden wird. Doch auch mit Erreichen der Zielkompressionsstellung wird die Anzeige des Schrittes S5 aufrechterhalten, um Nachkorrekturen möglichst einfach zu erlauben.

Da der Solldruck und die Referenzkontaktfläche über den Referenzflächenwert bekannt sind, lässt sich aus dem Kraftwert ablesen, ob der Solldruck und somit die Zielkompressionsstellung erreicht ist. Dies wird im Schritt S7 in einer Abbruchbedingung überprüft. Dabei ist es zum einen denkbar, dass aus dem aktuellen Kraftwert und dem Referenzflächenwert ein aktueller Druckwert ermittelt wird, der mit dem Solldruck verglichen wird, als auch zum anderen, dass aus dem Solldruck und dem Referenzflächenwert ein individueller Schwellwert für den Kraftwert hergeleitet wird, dessen Erreichen überwacht wird. Solange die Abbruchbedingung nicht erfüllt ist, wird die zweite Betriebsphase (Schritt S4) fortgesetzt.

Ist die Abbruchbedingung aber erfüllt, wird in Schritt S8 - unter fortwährender Anzeige der relativen Größe der Brust gemäß dem Referenzflächenwert zum Objektträger und/oder zur Kompressionsplatte - überprüft, ob eine manuelle Nachjustage gewünscht ist, beispielsweise, wenn die Brustgröße zu stark von der statistisch wahrscheinlichsten Annahme abweicht. Dann kann in einem Schritt S9 eine manuelle Anpassung der Kompressionskraft/Kompressionsstellung erfolgen, wobei parallel eine Einstellhilfe auf der Anzeigeeinrichtung ausgegeben wird.

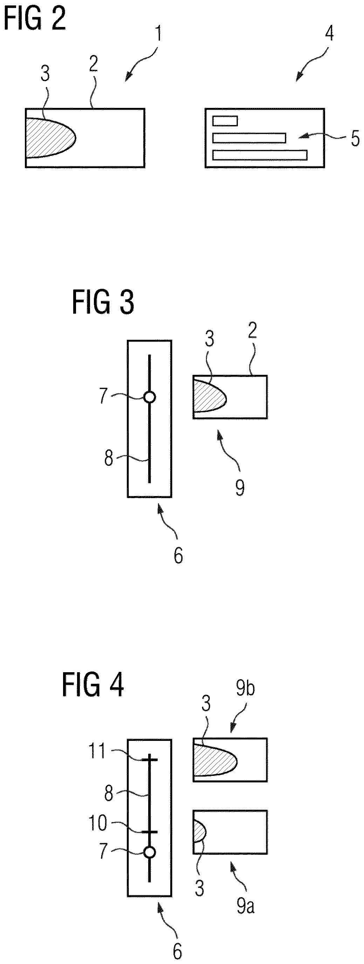

Dies sei im Hinblick auf Fig. 2, die eine erste mögliche Anzeige auf der Anzeigeeinrichtung zeigt, näher erläutert. Ein erstes Anzeigeelement 1 zeigt als Rechteck 2, das von einer Seite her mit einer stilisierten Brustgrafik 3 aufgefüllt ist, die Kontaktfläche gemäß Referenzflächenwert relativ zu der Kompressionsplatte und/oder dem Objektträger, welche bzw. welcher durch das Rechteck 2 symbolisiert wird. Das Anzeigeelement 1 wird während der zweiten Betriebsphase, mithin ab Bestimmung im Schritt S3, kontinuierlich angezeigt. Ist eine manuelle Anpassung der Kompressionsstellung erwünscht (vergleiche Schritt S8 und Schritt S9), wird als weiteres Anzeigeelement 4 eine Information 5 angezeigt, die beschreibt, wie die aktuelle Kompression für kleinere und/oder größere Untersuchungsobjekte anzupassen wäre, um den Solldruck zu erhalten.

Ein Anzeigekonzept gemäß dem Anzeigeelement 1 ist auch anderweitig bei der manuellen Einstellung einer geeigneten Kompressionsstellung zweckmäßig. So zeigt Fig. 3 in einer zweiten denkbaren Anzeige auf der Anzeigeeinrichtung ein Anzeigeelement 6, das die aktuelle Kompressionskraft (Marker 7 auf einer Skala 8) zeigt. Neben dem Marker 7 wird als weiteres Anzeigeelement 9 nun dargestellt, für welche Brustgröße (Kontaktfläche) sich bei dieser aktuellen Kompressionskraft der Solldruck ergibt. Somit erhält die einstellende Bedienperson einen intuitiv verständlichen Hinweis darauf, ob eine passende Kompressionsstellung gefunden wurde. Die Steuereinrichtung visualisiert bei der manuellen Wahl einer Kompressionsstellung also für den aktuellen Kraftwert, bei welcher relativen Fläche der Brust zu der Fläche der Kompressionsplatte/des Objektträgers der Solldruck gegeben ist.

Wie Fig. 4 zeigt, ist dies auch als statische Hinweisdarstellung möglich. Dort sind an der Skala 8 ein Minimalwert 10 und ein Maximalwert 11 markiert, denen jeweils ein Anzeigeelement 9a, 9b mit entsprechend ermittelten Brustgrafiken 3 zugeordnet ist. Gegebenenfalls können auch für weitere Referenzkräfte neben dem Minimalwert 10 und dem Maximalwert 11 entsprechende Anzeigeelemente eingeblendet werden.

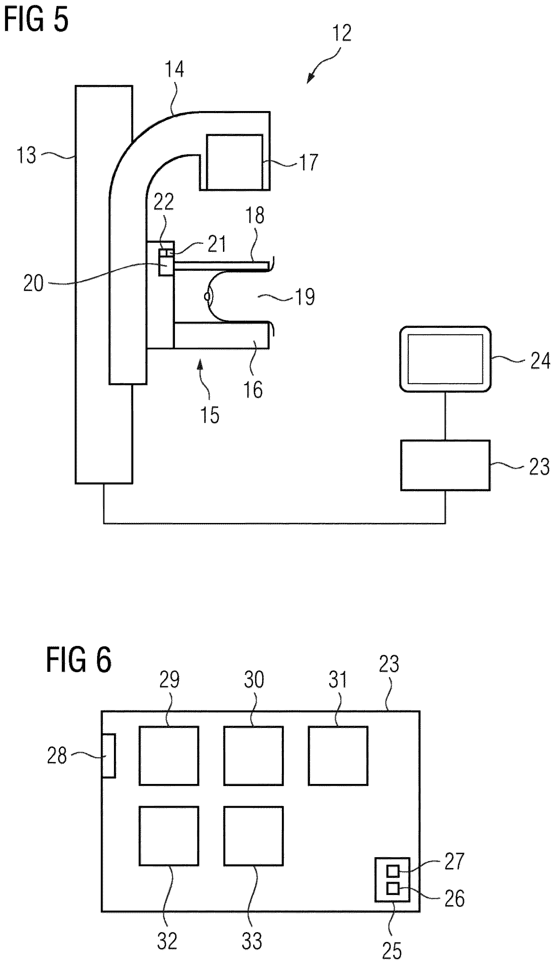

Fig. 5 zeigt eine Prinzipskizze einer erfindungsgemäßen Mammographieeinrichtung 12. Diese weist ein Stativ 13 auf, in dem höhenverstellbar eine Trägereinheit 14 geführt ist, die die Aufnahmeanordnung und die Trageanordnung der Mammographieeinrichtung 12 umfasst. Die Trägereinheit umfasst mithin einen Objektträger 15, der durch den Röntgendetektor 16 gebildet wird oder diesen zumindest umfasst. Der Röntgendetektor 16 kann Röntgenstrahlung eines insbesondere zur Tomosynthese beweglich gelagerten Röntgenstrahlers 17 empfangen. Zwischen dem Röntgenstrahler 17 und dem Objektträger 15/Röntgendetektor 16 ist eine Kompressionsplatte 18 einer Kompressionseinrichtung bewegbar angeordnet. Schematisch ist zur Verdeutlichung der Funktionsweise auch eine zwischen dem Objektträger 15 und der Kompressionsplatte 18 komprimierte Brust 19 angedeutet.

Eine automatische Bewegung der Kompressionsplatte 18 ist mittels eines Aktors 20 möglich. Zudem sind der Kompressionsplatte 18 ein Positionserfassungsmittel 21 sowie wenigstens ein Krafterfassungsmittel 22 zugeordnet.

Der Betrieb der Mammographieeinrichtung 12 wird durch eine Steuereinrichtung 23 gesteuert, die auch mit einer Anzeigeeinrichtung 24 verbunden ist. Die Steuereinrichtung 23 ist zur Durchführung des bezüglich Fig. 1 erläuterten Verfahrens ausgebildet. Hierzu weist die Steuereinrichtung 23 die in Fig. 6 näher gezeigte funktionale Struktur auf.

Der Übersichtlichkeit halber sind in Fig. 6 nur für das Verfahren relevante Komponenten gezeigt, wobei die Steuereinrichtung 23 selbstverständlich auch weitere Funktionseinheiten umfassen kann, beispielsweise eine Aufnahmeeinheit zur Steuerung des Aufnahmebetriebs.

Die Steuereinrichtung 23 umfasst ein Speichermittel 25, in dem vorliegend insbesondere die erste Zuordnungsvorschrift 26 und die zweite Zuordnungsvorschrift 27, beide als Look-up-Tabelle, gespeichert sind. Über eine Schnittstelle 28 werden neben anderen Daten ein aktueller Positionswert des Positionserfassungsmittel 21 und ein aktueller Kraftwert des Krafterfassungsmittels 22 entgegengenommen. Eine Steuereinheit 29 steuert die Bewegung bzw. Kraftausübung der Kompressionsplatte 18 über den Aktor 20, insbesondere gemäß dem Schritt S1 in der ersten Betriebsphase und gemäß dem Schritt S4 in der zweiten Betriebsphase. Eine Triggereinheit 30 überwacht gemäß dem Schritt S2, ob der Erstkontaktzeitpunkt erreicht ist. Eine Ermittlungseinheit 31 ist ausgebildet, aus dem Referenzhöhenwert gemäß Schritt S3 unter Nutzung der zweiten Zuordnungsvorschrift 27 den Referenzflächenwert zu ermitteln.

In einer Vorgabeeinheit 32 kann der Solldruck gemäß dem Schritt S5 ermittelt werden, wozu, wie beschrieben, die erste Zuordnungsvorschrift 26 eingesetzt wird. Schließlich ist auch eine Anzeigeeinheit 33 zur Erzeugung der verschiedenen Anzeigen, insbesondere gemäß Fig. 2 bis Fig. 4, vergleiche auch Schritte S6 und S9, vorgesehen.

Für die Überwachung der Abbruchbedingung ist vorliegend auch die Steuereinheit 29 zuständig (Schritt S7). Eine Benutzerinteraktionseinheit (nicht gezeigt), die die Anzeigeeinheit 33 umfassen kann, kann bei manueller Einstellung einer Kompressionsstellung, insbesondere auch bei einer Anpassung ausgehend von einer Zielkompressionsstellung, Benutzereingaben entgegennehmen und verarbeiten, insbesondere derart, dass die Steuereinheit 29 den Aktor 20 entsprechend ansteuern kann.

Unabhängig vom grammatikalischen Geschlecht eines bestimmten Begriffes sind Personen mit männlicher, weiblicher oder anderer Geschlechteridentität mit umfasst.

## Patentansprüche

1. Computerimplementiertes Verfahren zum Betrieb einer Mammographieeinrichtung (12), welche aufweist:
- einen Objektträger (15) für ein aufzunehmendes Untersuchungsobjekt,
- eine dem Objektträger (15) zugeordnete Kompressionseinrichtung mit wenigstens einer beweglichen Kompressionsplatte (18), der ein Aktor (20) zur Bewegung der Kompressionsplatte (18) zugeordnet ist,
- wenigstens ein Positionserfassungsmittel (21) zur Erfassung eines Positionswerts, der die aktuelle Position der beweglichen Kompressionsplatte (18) beschreibt, und wenigstens ein Krafterfassungsmittel (22) zur Erfassung eines Kraftwerts, der die durch die Kompressionsplatte (18) ausgeübte Kraft beschreibt, und
- eine Steuereinrichtung (23), die zum automatischen Einstellen einer Zielkompressionsstellung der beweglichen Kompressionsplatte (18) für ein Untersuchungsobjekt durch Ansteuerung des Aktors (20) in Abhängigkeit von dem Positionswert, dem Kraftwert und einem vorgegebenen Schwellwert ausgebildet ist,
wobei das Verfahren folgende Schritte zum automatischen Einstellen einer Kompression des Untersuchungsobjekts für einen Aufnahmevorgang aufweist:
- Ermitteln eines Referenzflächenwerts, der eine angenommene Kontaktfläche zwischen dem komprimierten Untersuchungsobjekt und der beweglichen Kompressionsplatte (18) in der Zielkompressionsstellung beschreibt, unter Verwendung des Kraftwerts und des Positionswerts zu wenigstens einem Referenzzeitpunkt,
- Komprimieren des Untersuchungsobjekts durch Ansteuerung des Aktors (20), bis eine Abbruchbedingung erfüllt ist, wobei die Abbruchbedingung das Erreichen eines vorgegebenen Solldrucks durch den Kraftwert bezogen auf den Referenzflächenwert beschreibt.

2. Verfahren nach Anspruch 1, **dadurch gekennzeichnet, dass** zur Überprüfung der Erfüllung der Abbruchbedingung
- ein den aktuellen Druck auf das Untersuchungsobjekt beschreibender aktueller Druckwert aus dem aktuellen Kraftwert und dem Referenzflächenwert ermittelt wird, und
- der aktuelle Druckwert mit dem als der Solldruck bereitgestellten Schwellwert verglichen wird, wobei die Abbruchbedingung erfüllt ist, wenn der Druckwert größer oder gleich dem Schwellwert ist,
oder
- ein vorgangsspezifischer Schwellwert für den Kraftwert aus dem Solldruck und dem Referenzflächenwert ermittelt wird und
- der aktuelle Kraftwert mit dem Schwellwert verglichen wird, wobei die Abbruchbedingung erfüllt ist, wenn der Kraftwert größer oder gleich dem Schwellwert ist.

3. Verfahren nach Anspruch 1 oder 2, **dadurch gekennzeichnet, dass** der Solldruck in Abhängigkeit wenigstens einer den Untersuchungsvorgang, insbesondere die Zieldimensionalität des zu ermittelnden Bilddatensatzes, beschreibenden Untersuchungsinformation vorgegeben wird.

4. Verfahren nach Anspruch 3, **dadurch gekennzeichnet, dass** der Solldruck für eine dreidimensionale Aufnahme niedriger vorgegeben wird als für eine zweidimensionale Aufnahme.

5. Verfahren nach einem der vorangehenden Ansprüche, **dadurch gekennzeichnet, dass** in Abhängigkeit des Verlaufs des Kraftwerts über den Positionswert wenigstens eine Eigenschaftsinformation des Untersuchungsobjekts ermittelt wird und der Solldruck in Abhängigkeit der Eigenschaftsinformation, insbesondere gemäß einer ersten Zuordnungsvorschrift (26), vorgegeben wird.

6. Verfahren nach Anspruch 5, **dadurch gekennzeichnet, dass** neben dem Verlauf auch der Referenzflächenwert in die Ermittlung der Eigenschaftsinformation eingeht.

7. Verfahren nach einem der vorangehenden Ansprüche, **dadurch gekennzeichnet, dass** als Referenzzeitpunkt der Erstkontaktzeitpunkt der Kompressionsplatte (18) mit dem Untersuchungsobjekt mittels wenigstens eines des wenigstens einen Krafterfassungsmittels (22) detektiert wird, wobei aus dem Positionswert zum Referenzzeitpunkt ein Referenzhöhenwert des Untersuchungsobjekts als Abstand zum Objektträger (15) ermittelt wird und aus dem Referenzhöhenwert der Referenzflächenwert ermittelt wird.

8. Verfahren nach Anspruch 7, **dadurch gekennzeichnet, dass** zur Ermittlung des Referenzflächenwerts aus dem Referenzhöhenwert eine statistisch ermittelte zweite Zuordnungsvorschrift (27), die einem Referenzhöhenwert eine statistisch wahrscheinlichste Referenzfläche als Referenzflächenwert zuordnet, verwendet wird.

9. Verfahren nach Anspruch 7 oder 8, **dadurch gekennzeichnet, dass** die Ansteuerung des Aktors (20) in zwei Betriebsphasen erfolgt, wobei die Kompressionsplatte (18) in der ersten Betriebsphase an das Untersuchungsobjekt, insbesondere mit konstanter Geschwindigkeit, herangefahren wird, und ab dem Referenzzeitpunkt in einer zweiten Betriebsphase mit insbesondere über die Zeit steigender Kompressionskraft zur Kompression des Untersuchungsobjekts weiterbewegt wird.

10. Verfahren nach einem der vorangehenden Ansprüche, **dadurch gekennzeichnet, dass** wenigstens eines des wenigstens einen Krafterfassungsmittels (22) als Kraftsensor die über die Kompressionsplatte (18) ausgeübte Kompressionskraft unmittelbar misst und/oder wenigstens eines des wenigstens einen Krafterfassungsmittels (22) eine Zeit, insbesondere ab einem Erstkontakt und/oder der zweiten Betriebsphase, misst und der Kraftwert aufgrund eines bekannten Ansteuerungsverlaufs mit der Zeit ermittelt wird.

11. Verfahren nach einem der vorangehenden Ansprüche, **dadurch gekennzeichnet, dass** die Mammographieeinrichtung (12) ferner eine Anzeigeeinrichtung (24) aufweist, die von der Steuereinrichtung (23) zur Visualisierung des ermittelten Referenzflächenwerts in Relation zu der Größe der Kompressionsplatte (18) und/oder des Objektträgers (15) angesteuert wird.

12. Verfahren nach Anspruch 11, **dadurch gekennzeichnet, dass** zur Visualisierung ein die Größe der Kompressionsplatte (18) und/oder des Objektträgers (15) anzeigendes Rechteck (2) gemäß der Form des Untersuchungsobjekts teilweise, den relativen Anteil des Referenzflächenwerts anzeigend, aufgefüllt wird.

13. Verfahren nach Anspruch 11 oder 12, **dadurch gekennzeichnet, dass**, insbesondere für einen manuellen Einstellvorgang, zusätzlich eine Information (5) angezeigt wird, die beschreibt, wie die aktuelle Kompression für kleinere und/oder größere Untersuchungsobjekte anzupassen wäre, um den Solldruck zu erhalten.

14. Verfahren nach einem der Ansprüche 11 bis 13, **dadurch gekennzeichnet, dass** die Steuereinrichtung (23) ferner dazu ausgebildet ist, für den aktuellen Kraftwert und/oder wenigstens eine Referenzkraft zu visualisieren, bei welcher relativen Fläche des Untersuchungsobjekts zu der Fläche der Kompressionsplatte (18) der Solldruck gegeben ist.

15. Mammographieeinrichtung (12), welche aufweist:
- einen Objektträger (15) für ein aufzunehmendes Untersuchungsobjekt,
- eine dem Objektträger (15) zugeordnete Kompressionseinrichtung mit wenigstens einer beweglichen Kompressionsplatte (18), der ein Aktor (20) zur Bewegung der Kompressionsplatte (18) zugeordnet ist,
- wenigstens ein Positionserfassungsmittel (21) zur Erfassung eines Positionswerts, der die aktuelle Position der beweglichen Kompressionsplatte (18) beschreibt, und wenigstens ein Krafterfassungsmittel (22) zur Erfassung eines Kraftwerts, der die durch die Kompressionsplatte (18) ausgeübte Kraft beschreibt, und
- eine Steuereinrichtung (23), die zum automatischen Einstellen einer Zielkompressionsstellung der beweglichen Kompressionsplatte (18) für ein Untersuchungsobjekt durch Ansteuerung des Aktors (20) in Abhängigkeit von dem Positionswert, dem Kraftwert und einem vorgegebenen Schwellwert ausgebildet ist,
wobei die Steuereinrichtung (23) zum automatischen Einstellen einer Kompression des Untersuchungsobjekts für einen Aufnahmevorgang aufweist:
- eine Schnittstelle (28) zum Empfang des Kraftwerts und des Positionswerts,
- eine Ermittlungseinheit (31) zum Ermitteln eines Referenzflächenwerts, der eine angenommene Kontaktfläche zwischen dem komprimierten Untersuchungsobjekt und der beweglichen Kompressionsplatte (18) in der Zielkompressionsstellung beschreibt, unter Verwendung des Kraftwerts und des Positionswerts zu wenigstens einem Referenzzeitpunkt, und
- eine Steuereinheit (29) zur Komprimieren des Untersuchungsobjekts durch Ansteuerung des Aktors (20), bis eine Abbruchbedingung erfüllt ist, wobei die Abbruchbedingung das Erreichen eines vorgegebenen Solldrucks durch den Kraftwert bezogen auf den Referenzflächenwert beschreibt.
